# EUROPEAN PATENT SPECIFICATION

(11) **EP 0 876 399 B1**
(45) Date of publication and mention of the grant of the patent: **24.04.2002**
(21) Application number: 97900735.8
(22) Date of filing: 16.01.1997
(51) Int. Cl.: C07K 14/415, A61K 38/16, A61K 38/56

(54) **PEPTIDES AND USES THEREOF FOR THERAPY OF CELIAC DISEASES**
PEPTIDE UND IHRE VERWENDUNG IN DER THERAPIE VON KRANKHEITEN DES ZÖLIAKIE-FORMENKREISES
PEPTIDES ET LEURS UTILISATIONS DANS LA THERAPIE DE MALADIES COELIAQUES

(30) Priority: 26.01.1996 IT RM960052
(43) Date of publication of application: 11.11.1998
(73) Proprietor: ISTITUTO SUPERIORE DI SANITA', 00161 Roma (IT)
(72) Inventor: DE VINCENZI, Massimo, I-00161 Roma (IT)
(74) Representative: de Simone, Domenico
(86) International application number: IT9700007
(87) International publication number: WO9727217

(56) References cited:
- WO-A-94/26774
- US-A- 4 826 765
- TOXICOLOGY, vol. 96, no. 1, 1995, pages 29-35, XP000672200 M. DE VINCENZI ET AL.: "Agglutinating activity of wheat gliadin peptide fractions in coeliac disease" cited in the application
- BIOCHEMICAL AND BIOPHYSICAL RESEARCH COMMUNICATIONS, vol. 121, no. 1, 15 June 1984, DULUTH, MINNESOTA US, pages 428-433, XP000673546 S. AURICCHIO ET AL.: "Agglutinating Activity of Gliadin-Derived Peptides from Bread Wheat: Imlications for Coeliac Disease Pathogenesis"

## Description

The invention concerns peptides and uses thereof for the therapy of celiac syndrome, having an amino acid sequence derived from durum wheat proteins. Said peptides exert their protective activity against the toxicity of cereals for celiac syndrome affected subjects.

The celiac syndrome is an enteropathy which can affect genetically susceptible subjects (around 3‰) when wheat containing food is included in the diet. Besides of wheat also rye, barley and oats result to be toxic for said subjects. Symptoms comprise diarrhoea and other malabsorption symptoms, including total atrophy of intestinal mucosa.

It is known that in humans said pathologic alterations are produced by the action on the intestinal mucosa of digestion products of wheat gluten and, in particular, by the 70% ethanol soluble gluten protein fraction. Said protein fraction, generally named as "prolamin" (in wheat, in particular, it is named as "gliadin"), is present in several cereals in different proportions and is composed of numerous proteins with different molecular weights, and having an high glutamine (one glutamine residue every three amino acids) and proline (a proline residue every seven amino acids) content, and a low ionic strength (due to few residues able to ionise in solution).

The author of the instant invention has been performing experiments by means of tissue and cell cultures with peptide mix obtained by sequential proteolytic pepsin and trypsin in vitro digestion of cereal-extracted prolamin, by miming the digestive process of proteins in the gastrointestinal apparatus. The aim is to clarify the composition of toxic peptides obtained by different prolamins and to establish if some particular wheat species and/or cultivar are better tolerated by celiac subjects.

Experiments are essentially based on three biologic systems:
- bioptic intestinal fragment cultures from celiac subjects in healthy phase;
- rat intestinal fragment cultures;
- agglutination test with erythroid cell lines K562 (S).

The author has shown that digestion products of durum wheat extracted gliadin are non toxic, or at least are toxic in a very lower extent with respect to soft wheat extracted gliadin. As a matter of fact gliadin peptides from durum wheat: a) are not toxic for in vitro celiac bioptic cultures (Ped. Res. 16, 1004, 1983); b) do not inhibit the 17 day rat fetal gut development and morphogenesis (Ped. Res. 18, 1372, 1984); c) are not able to agglutinate K562(S) cells (Biochem. Biophys. Res. Comm. 121, 428, 1984), d) are not cytotoxic on either Hep-2 and MCR-5 cell lines (Toxicol. Lett. 13, 1255, 1983), or CaCo-2 cell line (Toxicology in vitro, 9, 251, 1995).

It has been believed that the different behaviour of durum wheat could be due to the tetraploid wheat (namely durum wheat varieties) low capacity of synthesising the peptide fraction responsible of the pathologic status in celiac subjects (Ped. Res. 22, 102, 1987). However, a peptide fraction having a comparable amount and toxic activity is present also in durum wheat. The activity of said fraction, in the agglutination test with K562 (S) cells, is inhibited by another peptide fraction of durum wheat (Toxicology, 96, 29, 1995).

The author of the instant invention has now identified a peptide which has an inhibiting activity for toxic gliadin induced K562 cell agglutination, thus representing an advantageous mean for the therapy and diagnosis of celiac or related disease affected subjects.

A durum wheat peptide mass spectrometry analysis evidentiates a 10 aminoacid peptide having the sequence: QQPQDAVQPF. Said peptide at a 2 x 10⁻³ mM concentration is able to protect K 562 (S) cells, against the action of any of cereals known to be toxic for celiac subjects: soft wheat, rye, barley and oats. Moreover the author was able to identify a five aminoacid "core" which is necessary to protect K 562 (S) cells.

The in vitro agglutination test with K 562 (S) cells is very sensitive to evaluate the toxicity of either cereal proteins or digestion product peptides thereof which are produced in the gut. At the moment a 100% correlation exists between the in vivo or in vitro cereal toxicity and the agglutinating activity. results are disclosed in the following references: (Biochem. Biophys. Res. Comm., 121, 428, 1984; Pediatric Res., 18, 189a, 1984; J. of Pediatric Gastr. and Nutr., 4, 923, 1985; Gastroenterology, 92, 1368, 1987; Gastroenterology, 92, 1301, 1987; Pediatric Res. 22, 107,1987; Pediatric Res., 24, 233, 1988; Pediatric Res., 24, 412, 1988; Gastroenterology, 99, 977, 1990; Gastroenterology, 99, 1668, 1990; Ital. J. Gastroenterology 24, 397, 1992; ATLA, 21, 43, 1993; Gastroenterology 104, 88, 1983; ATLA, 22, 116, 1994; ATLA, 22 (6) 50, 1994; Toxicology 96, 29, 1995; J. Scand. Gastroenterol. (in press).

"Celiac syndrome related pathologies" are to be intended: a) immunological diseases as diabetes mellitus, hyperthyroidism, Addison syndrome, recurrent pericarditis, myasthenia gravis, thrombocytopenic purpura, thrombocytopenic vasculitis, autoimmune haemolytic anaemia, biliary cirrhosis, chronic hepatitis, connective diseases, Sjogren syndrome, intestinal inflammation diseases, IgA nephropathy, atopy, milk protein intolerance; b) neurologic and psychiatric pathologies as progressive encephalopaty, polyneuropathy, cerebral atrophy dementia, autism and schizophrenia, leukoencephalopaty and axonal neuropathy, epilepsy with posterior cerebral calcifications; c) cystic fibrosis, Down syndrome, Hartnup disease, congenital cardiopathies, neoplasia.

It is therefore an object of the invention a protein compound having a sequence comprised in the proteins of durum wheat and being not toxic for celiac subjects characterised in having an aminoacid sequence comprised in the following sequence: QQPQDAVQPF, or substantially homologous so to retain the functional activity. Preferably the compound comprises at least the following amino acid sequence: QQPQD, more preferably the sequence: QQPQDA, most preferably the sequence: QQPQDAV, even more preferably the sequence QQPQDAVQ, even more preferably the sequence QQPQDAVQP.

The compound may be produced synthetically or purified from durum wheat.

It is a further object of the invention a protein compound according to any of previous claims for use in medicine and diagnosis, for use in the therapy of celiac and celiac related diseases.

It is a further object of the invention a pharmaceutical composition comprising in acceptable and effective dosages the compound of the invention, to be used for celiac and celiac related disease therapy.

It is a further object of the invention a pharmaceutical composition comprising in acceptable and effective dosages the compound of the invention, to be used for celiac and celiac related disease diagnosis.

It is a further object of the invention a method for the identification of molecules having a protective activity towards celiac and celiac related disease affected subjects comprising the steps as follows:
- incubating cereal prolamin fraction sensitive cells with an effective amount of said cereal prolamin fraction and of the compound of the invention; and measuring the inhibiting activity of the compound of the invention in respect of the agglutination activity of said cereal prolamin fraction for said prclamin fraction sensitive cells. Preferably said cells are K562 cells.

The invention will be now described for illustrating but not limiting purposes, by reference to the following figures wherein:
- figure 1 represents the elution curve on a Sepharcse6B/mannane column of a gliadin peptic digest;
- figure 2 represents the elution curve on a Biogel-P30 column of the fraction 3 of fig. 1;
- figure 3 represents a mass spectrometry curve of the fraction 1D of fig. 2;
- figure 4 represents a mass spectrometry curve of the synthetic peptide 1157;
- figure 5 represents phase contrast microscope photographs of K562 cells: control (A), treated with the soft wheat gliadin fraction (B), treated with both the soft wheat gliadin fraction and pepride 1157 (C).

### a) Gliadin extraction and preparation of the enzymatic digest

Durum wheat 70% ethanol extracted gliadins (Adamello variety) were subjected to pepsin and trypsin enzymatic hydrolysis for 6 hrs. The mix of peptides was heated at 100°C for 30', to inactivate the remaining enzymatic activities.

### b) Affinity chromatography

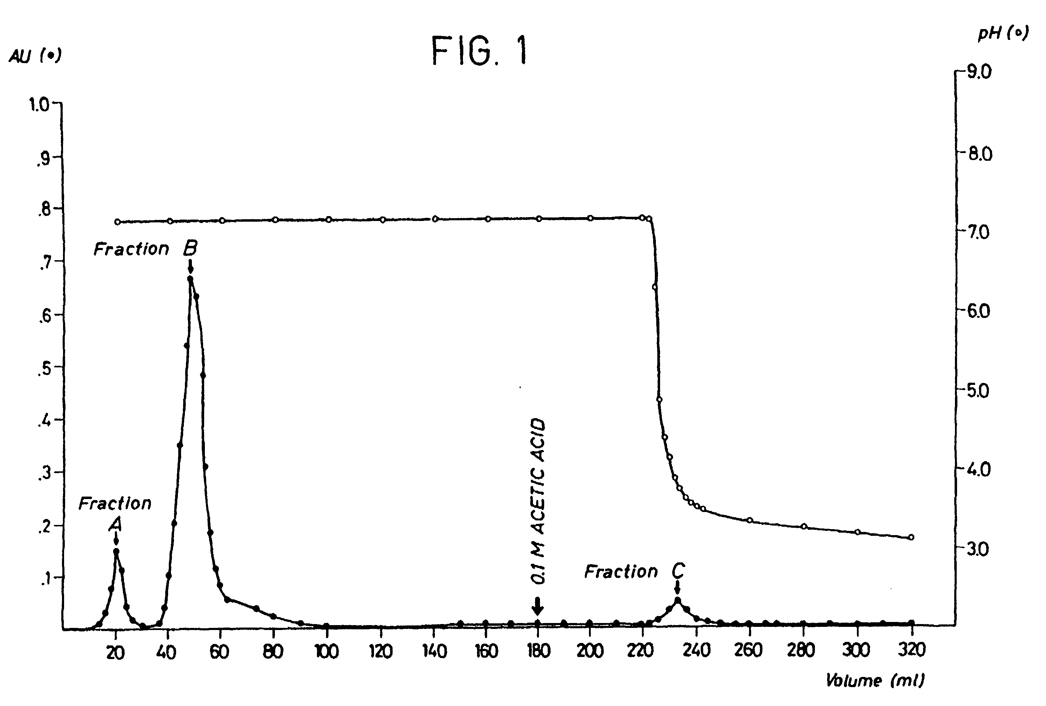

Approximately 50 mg of gliadin digest were loaded on a column (Sepharose 6B/Mannane) 35 x 5 cm, equilibrated with ammonium acecate buffer. Fractions A e B have been eluted with the washing buffer, whereas fraction C was eluted with acetic acid 0.1 M (Fig 1). This fraction (appr. 1.5 % of the total loaded material) unlike the inactive total starting mix, showed an agglutination activity similar to fraction C as obtained from other toxic cereals.

Surprisingly fraction B, approximately 90-95% of the total digest, is able to inhibit the fraction C induced biological activity. Moreover said fraction B, still biological activity. Moreover said fraction B, still heterogeneous, protected cells with respect to the toxic activity of different cereals. Said data show that a toxic fraction is present also in the durum wheat, and that its activity is inhibited by other peptides from the same durum wheat.

### c) Biogel-P30 chromatography

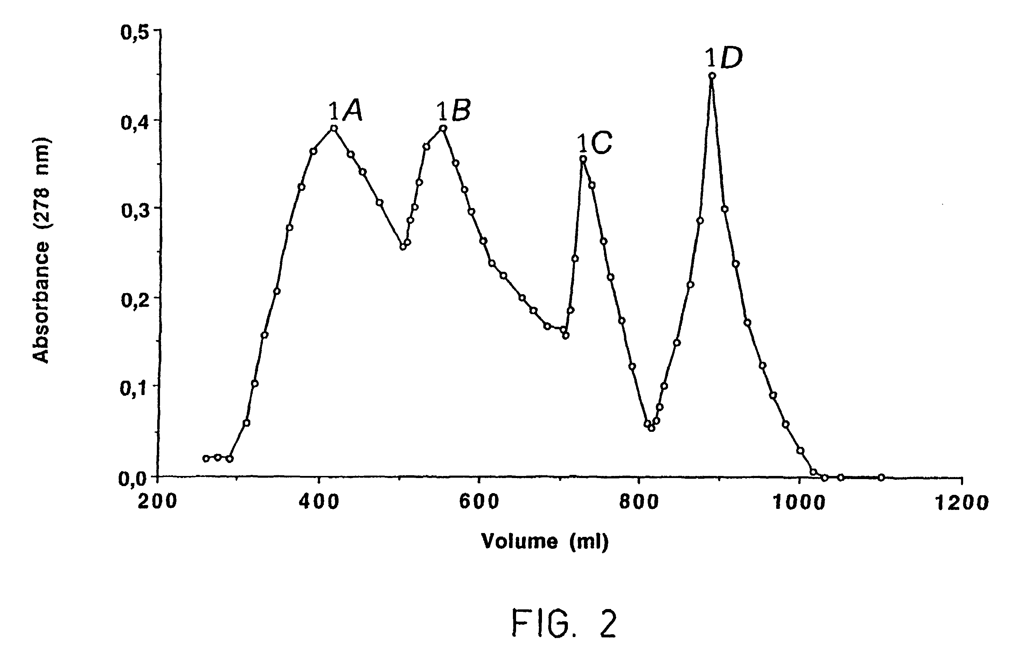

Fraction B obtained by affinity chromatography was further fractionated on a Biogel-P30 column (3 cm x 170 cm) (Fig.2). The protective activity of the four eluted fractions was evaluated with the agglutination test. Fractions 1A, 1B, 1C were unable to protect K562 (S) cells with respect to the toxic activity of prolamin peptides. On the contrary fraction 1D, assayed at 70 mg/l, showed an high protective activity for celiac toxic cereal prolamins.

### d) Mass spectrometry

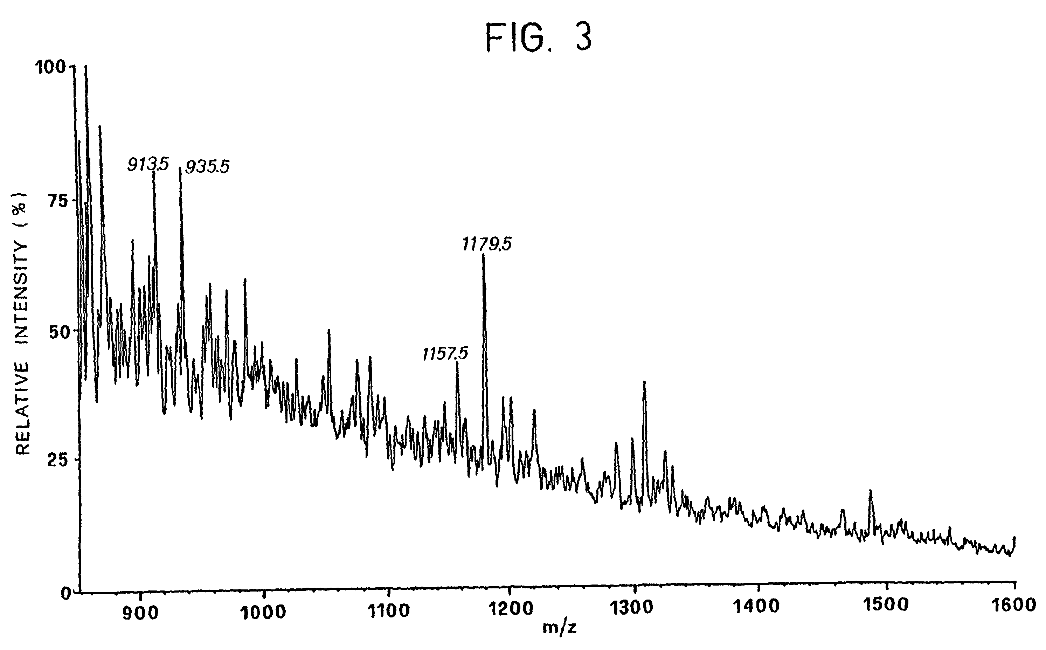

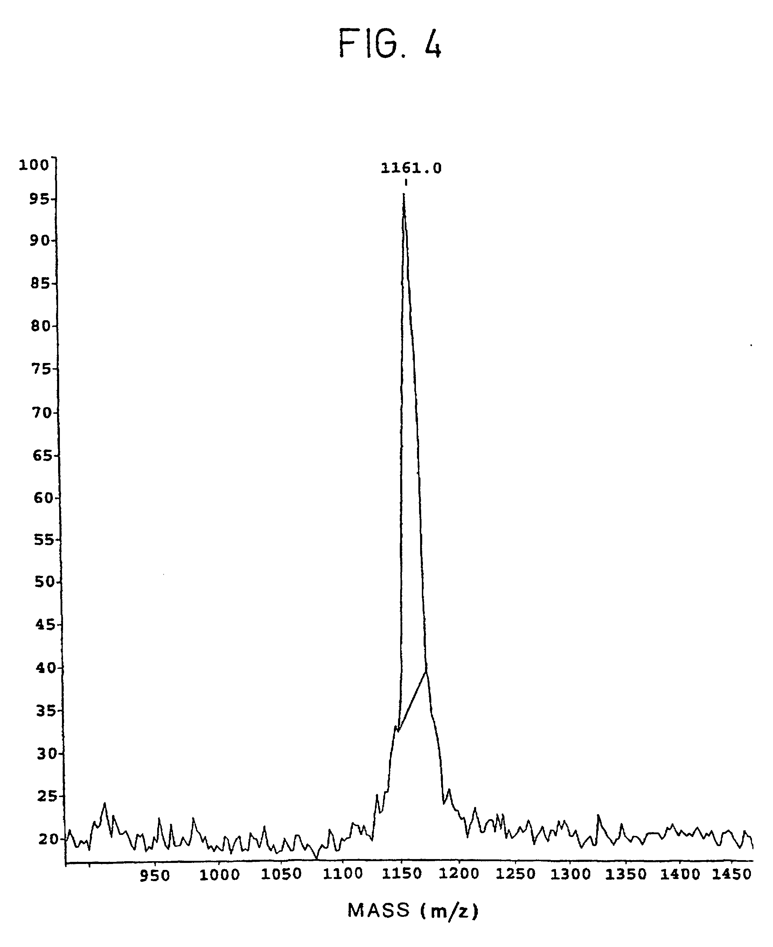

Fraction 1D, dissolved in CH₃CN/H₂O, 60/40 with TFA 0.1 % and ammonium acetate 30 mM, was examined by means of mass spectrometry with continuous infusion, utilising a pump at a 10 ml/m speed. Fig. 3 shows the mass spectrum of the fraction 1D enzymatic digest, wherein a peptide having a molecular weight of approximately 1157 is present. The peptide aminoacid sequence is as follows: QQPQDAVQPF. The peptide, named as 1157, was synthesised with an automatic synthesizer (Applied Biosystems 431A), having a purity higher than 98% (Fig.4) and assayed with K562 cells. Deletion peptides were also synthesised and tested as for peptide 1157.

### e) Cells and agglutination test

Cells were K562(S) cells, an established cell line, isolated by Lozzio from a chronic leukaemia affected subject during an acute phase. Said cells are considered to be proerythroblasts able to undergo in vitro a differentiation process similar to the in vivo process. Cells were grown in RPMI 1640 medium with fetal serum 10%, glutamine 2mM, penicillin and streptomycin. K562 (S) cells were centrifuged, washed three times with buffer at 100 x 10⁶ cells/ml. For the agglutination test, 25 µl of the cell suspension was added to the wells of a microtiter plate with 25 µl of buffer containing the tested peptides. Increasing amounts of the compound to be tested, obtained with sequential 1:1 buffer dilutions, were added to the cells. A microscope observation was performed 30' after the room temperature incubation.

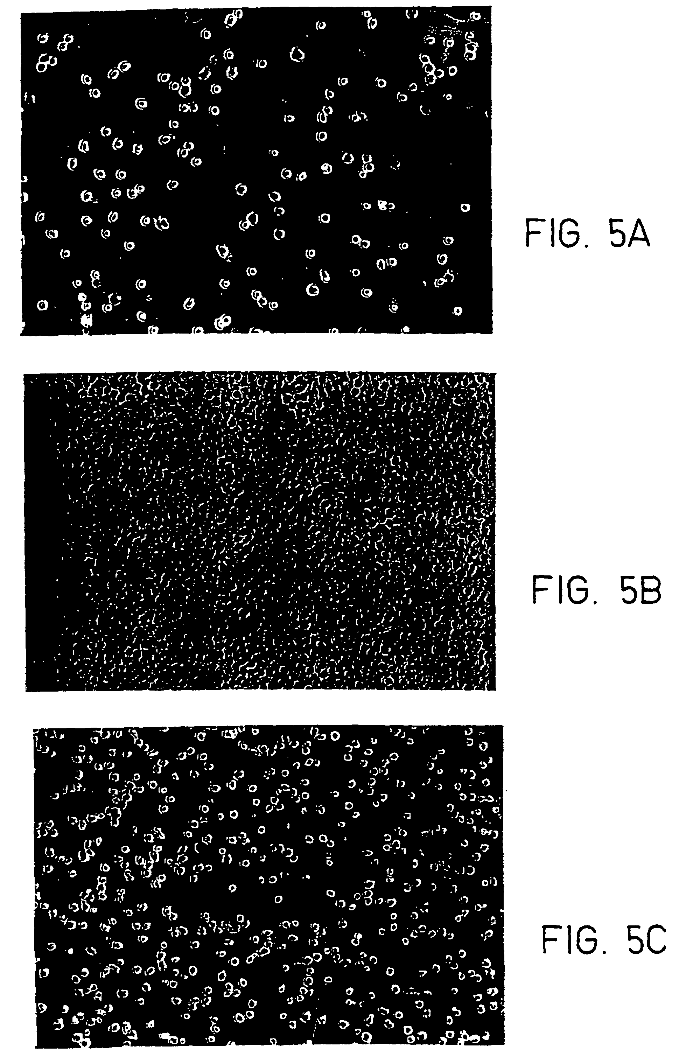

Fig. 5A shows the control cells; Fig. 5B shows the agglutination induced by one of toxic cereals (soft wheat); Fig. 5C shows the protective activity of peptide 1157 with respect to the soft wheat toxicity.

Table 1 show the amino sequence of deletion peptides and of a paptide with a glycin residue addition (9).

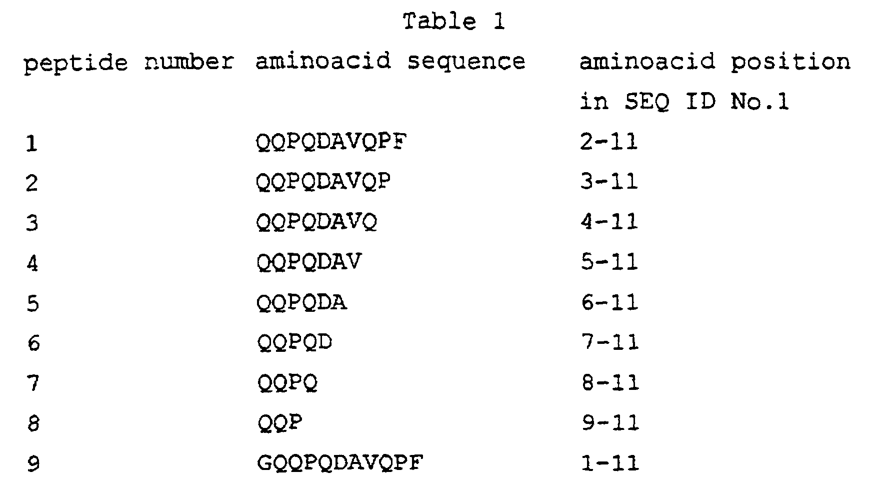

As shown in Table 2 only peptides 1 to 6 were able to inhibit agglutination of K 562 (S) cells. Peptides from 7 to 9 were inactive.

Table 3 shows the effect of the same peptides on the agglutinating activity of prolamins from different cereals on K 562 (S) cells.

**Table 2 -**

| Effects of peptides from durum wheat gliadin on the agglutinating activity of fragments from A-gliadin on K562(S) cells | | | | | | | |
|---|---|---|---|---|---|---|---|
| fragments from A-gliadin | MAC 10⁻³ (mM)^{a} | MIC 10⁻³ (mM)^{b} Inhibitors (peptides from durum wheat as in Table 1) | | | | | |
| | | 1 | 2 | 3 | 4 | 5 | 6 |
| 31-43 | 8.5 | 2.8 | 3.2 | 3.6 | 2.12 | 2.43 | 2.7 |
| 44-55 | 36 | 1.4 | 1.6 | 1.8 | 2.12 | 2.43 | 1.4 |

| | | | | | | | |
|---|---|---|---|---|---|---|---|
| ^{a} The minimal concentration in the agglutination suspension required to agglutinate 100% of total cells. | | | | | | | |
| ^{b} Calculation of the minimal concentration of inhibitors required to completely inhibit cell agglutination induced by fragments 31-43 and 44-55 from A-gliadin, tested at 2.18 mM and 2.35 mM respectively. | | | | | | | |

**Table 3**

| Effects of peptides from durum wheat gliadin on the agglutinating activity of prolamins on K562(S) cells | | | | | | | |
|---|---|---|---|---|---|---|---|
| Prolamins | MAC 10⁻³ (mM)^{a} | MIC 10⁻³ (mM)^{b} Inhibitors (peptides from durum wheat as in Table 1) | | | | | |
| | | 1 | 2 | 3 | 4 | 5 | 6 |
| rye | 146 | 0.7 | 0.8 | 0.9 | 1.1 | 1.2 | 1.35 |
| barley | 146 | 0.7 | 0.8 | 0.9 | 1.1 | 1.2 | 1.35 |
| oats | 292 | 0.35 | 0.4 | 0.5 | 0.6 | 0.6 | 0.7 |

| | | | | | | | |
|---|---|---|---|---|---|---|---|
| ^{a} The minimal concentration in the agglutination suspension required to agglutinate 100% of total cells. | | | | | | | |
| ^{b} The minimal concentration of peptides required to completely inhibit cell agglutination induced by a prolamin concentration six fold higher than the minimal concentration necessary to agglutinate all of cells. | | | | | | | |

The invention was described for illustrative but not limitative purpose, according to preferred embodiments, but it is to be intended that modifications may be apported by the expert in the field without to be out of the scope of the following claims.

## Claims

1. Protein compound having a sequence comprised in the proteins of durum wheat and being not toxic for celiac subjects **characterised in** having an aminoacid sequence comprised in the following sequence: QQPQDAVQPF, or substantially homologous so to retain the functional activity.

2. Protein compound according to claim 1 comprising at least the following amino acid sequence: QQPQD.

3. Protein compound according to claim 2 comprising at least the following amino acid sequence: QQPQDA.

4. Protein compound according to claim 3 comprising at least the following amino acid sequence: QQPQDAV.

5. Protein compound according to claim. 4 comprising at least the following amino acid sequence: QQPQDAVQ.

6. Protein compound according to claim 5 comprising at least the following amino acid sequence: QQPQDAVQP.

7. Protein compound according to any of previous claims for use in medicine and diagnosis.

8. Protein compound according to claim 7 for use in the therapy of celiac and celiac related diseases.

9. Pharmaceutical composition comprising in acceptable and effective dosages the compound according to any of claims 1-6, to be used for celiac and celiac related disease therapy.

10. Pharmaceutical composition comprising in acceptable and effective dosages the compound according to any of claims 1-6, to be used for celiac and celiac related disease diagnosis.

11. Method for the identification of molecules having a protective activity towards celiac and celiac related disease affected subjects comprising the steps as follows:
- incubating cereal prolamin fraction sensitive cells with an effective amount of said cereal prolamin fraction and of the compound according to any of claims 1-6; and
- measuring the inhibiting activity of said compound respect to the agglutination activity of said cereal prolamin fraction for said prolamin fraction sensitive cells.

12. Method for the identification of molecules having a protective activity towards celiac and celiac related disease affected subjects according to claim 11 wherein said cells are K562 cells.

## Patentansprüche

1. Proteinverbindung, die eine in Protein von Hartweizen vorhandene Sequenz aufweist und fuer zöliakiesche Individuen nicht toxisch ist, **dadurch gekennzeichnet, dass** sie eine Aminosäurensequenz in der Reihenfolge: QQPQDAVQPF oder wesentlich homologe enthält, so dass sie funktionelle Wirkung ausübt.

2. Proteinverbindung nach Anspruch 1, die mindestens die folgende Aminosäurensequenz QQPQD aufweist.

3. Proteinverbindung nach Anspruch 2, die mindestens die folgende Aminosäurensequenz QQPQDA aufweist.

4. Proteinverbindung nach Anspruch 3, die mindestens die folgende Aminosäurensequenz QQPQDAV aufweist.

5. Proteinverbindung nach Anspruch 4, die mindestens die folgende Aminosäurensequenz QQPQDAVQ aufweist.

6. Proteinverbindung nach Anspruch 5, die mindestens die folgende Aminosäurensequenz QQPQDAVQP aufweist.

7. Proteinverbindung nach je einem der vorhergehenden Ansprüche zur Anwendung in Medizin und Diagnosis.

8. Proteinverbindung nach Anspruch 7, zur Anwendung in der Therapie von Zöliakiesche und damit verbundenen Krankheiten.

9. Pharmazeutische Zusammensetzung, die in einer verträgbaren und wirksamen Dosierung die Verbindung nach je einem der Ansprüche 1 bis 6 enthält, zur Anwendung für die Therapie von zöliakieschen oder damit verbundenen Krankheiten.

10. Pharmazeutische Zusammensetzung, die in verträgbaren und wirksamen Dosierung die Verbindung nach je einem der Ansprüche 1 bis 6 enthält, zur Anwendung für die Diagnose von zöliakieschen oder damit verbundenen Krankheiten.

11. Verfahren zur Identifizierung von Molekulen, die eine Schutzwirkung gegenüber den mit den zöliakieschen un damit verbundenen Krankheiten behafteten Individuen aufweisen; mit folgenden Verfahrensschritten:
- Inkubation von auf Getraide-Prolaminfraktion empfindlichen Zellen mit einer wirksamen Menge der vorgenannten Getreide-Prolaminfraktion und der Verbindung nach den Ansprüchen 1 bis 6, und
- Messung der Hemmwirkung der vorgenannten Verbindung gegenüber der Agglutinationswirkung der vorgenannten Getreide - Prolaminfraktion auf die vorgenannte Prolaminfraktion empfindlichen Zellen.

12. Verfahren zur Identifizierung von Molekülen, die eine Schutzwirkung gegenüber den mit den zöliakieschen und damit verbundenen Krakheiten behafteten Individuen aufweisen nach Anspruch 11, worin die genannten Zellen K562-Zellen sind.

## Revendications

1. Composé protéique ayant une séquence contenue dans le protéines de blé dur et n'étant pas toxique aux sujets coeliaques, **caractérisé en ce qu'**il comprend une sequence aminoacida contenue dans la suivante séquence QQPDAVQPF ou sensiblement homologue, de façon à retenir l'activité fonctionnelle.

2. Composé protéique selon la revendication 1 comprenant au moins la suivante séquence des aminoacides: QQPQD.

3. Composé protéique selon la revendication 2 comprenant au moins la suivante séquence des aminoacides: QQPQDA.

4. Composé protéique selon la revendication 3 comprenant au moins la suivante séquence des aminoacides: QQPQDAV.

5. Composé protéique selon la revendication 4 comprenant au moins la suivante séquence des aminoacides: QQPQDAVQ.

6. Composé protéique selon la revendication 5 comprenant au moins la suivante séquence des aminoacides: QQPQDAVQP.

7. Composé protéique selon une quelconque des revendications précédentes pour l'usage en médicine et diagnostic.

8. Composé protéique selon la revendication 7 pour l'usage dans la therapie des meladies coeliaques et correlatives.

9. Composition pharmaceutiques comprenant un dosage acceptable et efficace le composé selon une quelconque des revendications 1 à 6 pour être utilisée dans la therapie des maladies coeliaques et correlatives.

10. Composition pharmaceutiques comprenant un dosage acceptable et efficace le composé selon une quelconque des revendications 1 à 6 pour être utilisée dans la diagnose des maladies coeliaques et correlatives.

11. Procédé pour l'identfication de molecules ayant une action protective au regard de suject atteints de maladie coeliaque et correlative, comprenants les phases suivantes:
- incubation des cellules sensibles à la fraction céreale de prolamine avec une quantité efficace de ladite fraction céréale de prolamine et du composé selon une quelconque des revendications 1 a 6; et
- mesurage de l'activité d'inhibition dudit composé à l'égard de l'activité d'agglutination de ladite fraction céréale de prolamine pour lesdites cellules sensible à ladite fraction de prolamine.

12. Procédé pour l'identfication de molecules ayant une action protective au regard de suject atteints de maladie coeliaque et correlative selon la revendication 11, dans lequel lesdites cellules sont des cellules K562.


### SEQUENCE LISTING

(1) GENERAL INFORMATION:
   (i) APPLICANT:
      (A) NAME: Istituto Superiore di Sanita'
      (B) STREET: viale Regina Elena
      (C) CITY: Roma
      (E) COUNTRY: ITALY
      (F) POSTAL CODE (ZIP): 00161
   (ii) TITLE OF INVENTION: Peptides and uses thereof for therapy of celiac diseases
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (xi) SEQUENCE DESCRIPTION: SEQ ID NO: 1:
   Gly Gln Gln Pro Gln Asp Ala Val Gln Pro Phe
   1               5                   10